# EUROPÄISCHE PATENTANMELDUNG

(11) **EP 4 091 577 A1**
(43) Veröffentlichungstag der Anmeldung: **23.11.2022**
(21) Anmeldenummer: 22173858.6
(22) Anmeldetag: 17.05.2022
(51) Int. Cl.: A61C 13/00, A61C 13/08, A61K 6/818, A61K 6/887, A61K 6/16, A61K 6/60, A61K 6/76, C04B 41/00, C04B 41/50, C04B 41/85, C04B 35/486, C04B 111/00, C04B 111/80

(54) **DENTALE OPAKER-ZUSAMMENSETZUNG**

(30) Priorität: 20.05.2021 EP 21175082
(71) Anmelder: Ivoclar Vivadent AG, 9494 Schaan (LI)
(72) Erfinder: Serban, Corina, 88131 Lindau (DE); Rothbrust, Frank, 6822 Röns (AT); Ritzberger, Christian, 9472 Grabs (CH); Ott, Katja, 9492 Eschen (LI); Krolikowski, Sebastian, 8853 Lachen (CH); Stournari, Vasiliki-Kallirroi, 9470 Buchs (CH); Wildner, Christin, 7303 Mastrils (CH)
(74) Vertreter: Uexküll & Stolberg

(57) **Zusammenfassung**

Die Erfindung betrifft eine dentale Opaker-Zusammensetzung, mit der die Opazität von Zirkonoxidkeramiken erhöht werden kann. Darüber hinaus betrifft die Erfindung ein Verfahren zur Herstellung einer dentalen Restauration, bei dem unter Verwendung der Opaker-Zusammensetzung die Opazität der Zirkonoxidkeramik erhöht wird.

## Beschreibung

Die Erfindung betrifft eine dentale Opaker-Zusammensetzung, mit der die Opazität in dentalen Oxidkeramiken und insbesondere Zirkonoxidkeramiken erhöht werden kann.

Zirkonoxidkeramiken finden wegen ihrer vorteilhaften mechanischen Eigenschaften verbreitet Anwendung bei der Herstellung von dentalen Restaurationen. Als Ausgangsmaterial für dentale Restaurationen werden insbesondere Zirkonoxidmaterialien eingesetzt, die auf mit Y₂O₃ stabilisierten tetragonalen Polykristallen basieren.

Die Herstellung von dentalen Restaurationen aus Zirkonoxid, wie auch in der WO 2018/115529 A1 beschrieben, umfasst typischerweise zwei thermische Verdichtungsschritte, die durch einen Formgebungsschritt getrennt sind. Die Zirkonoxid-Ausgangsmaterialien werden dabei in der Regel zunächst gegossen oder unter Verwendung von Druck mechanisch verdichtet und dann zu einem intermediären offenporigen Zustand vorgesintert, um einen Rohling herzustellen. Dieser Rohling eignet sich für eine Formgebung oder Vorformgebung, z.B. durch maschinelle Bearbeitung in einem CAD/CAM-Verfahren. Der geformte Rohling kann dann in einem weiteren Sinterschritt einer abschließenden thermischen Verdichtung unterzogen werden.

Bei dentalen Restaurationen werden nicht nur hohe Anforderungen an die mechanischen Eigenschaften gestellt. Es ist auch von besonderer Wichtigkeit, das komplexe äußere Erscheinungsbild von natürlichen Zähnen naturgetreu zu imitieren. Dabei gilt es, sowohl die Transluzenz als auch die Farbgebung mit den jeweiligen Verläufen und 3D-Effekten nachzuahmen. Besonders für dentale Restaurationen aus Zirkonoxid stellt dies eine große Herausforderung dar, weshalb gerade im Bereich der Frontzähne andere Materialien, wie z.B. Lithiumsilikat-Glaskeramiken, trotz deren häufig dem Zirkonoxid unterlegenen mechanischen Eigenschaften bevorzugt werden.

Es kann in der zahnärztlichen Praxis vorkommen, dass eine Zirkonoxid-Restauration mit hoher Transluzenz auf ein dunkles Implantat, wie z.B. ein Titangerüst, oder einen verfärbten präparierten Zahnstumpf aufgebracht werden soll. Wegen der hohen Transluzenz der dentalen Restauration kann es dabei zu unerwünschten optischen Effekten kommen, bei denen das dunkle bzw. verfärbte Material durch die dentale Restauration sichtbar ist. Dieses Problem tritt insbesondere dort auf, wo Zirkonoxid-Restaurationen mit dünnen Wandstärken verwendet werden sollen, wie z.B. in zervikalen Bereichen von dentalen Restaurationen.

Es sind verschiedene Verfahren bekannt, um zu verhindern, dass dunkle Materialien durch eine Zirkonoxid-Restauration hindurch sichtbar sind.

Beispielsweise wird in aufwendigen Verfahren zunächst ein Gerüst aus opakem Zirkonoxid hergestellt, das dann mit einer Glaskeramik verblendet und gegebenenfalls glasiert wird.

Es ist auch möglich, die Sichtbarkeit eines sich unter der Zirkonoxidkeramik befindlichen Materials mithilfe von dentalen Opakern wie Infiltrationslösungen zu reduzieren oder gänzlich zu verhindern. Dazu wird die vorgesinterte dentale Restauration mit der Infiltrationslösung in Kontakt gebracht, indem die Infiltrationslösung auf die Restauration aufgetragen wird oder die Restauration in die Lösung eingetaucht wird. Die Infiltrationslösung bewirkt in der dentalen Restauration typischerweise eine weiße Färbung und eine Erhöhung der Opazität, wodurch das Durchscheinen der dunklen Materialien durch die Zirkonoxid-Restauration verhindert werden kann.

In der Regel werden die Infiltrationslösungen auf die Innenseite, d.h. in das Lumen, der dentalen Restauration aufgetragen. Das Auftragen von Infiltrationslösungen auf die Außenseite von dentalen Restaurationen ist nachteilig, weil es zu unterwünschten Wechselwirkungen mit dort aufgetragenen bzw. aufzutragenden Färbelösungen kommen kann oder die Verwendung von Färbelösungen vollständig verhindert wird.

Die WO 2013/170705 A1 offenbart eine Färbelösung, die eine Kombination von mindestens zwei Arten von Färbeionen ausgewählt aus der Gruppe bestehend aus den Ionen von Pr, Er, Ce und Nd sowie Lösungsmittel und ein Additiv enthält. Wenn diese Färbelösung auf eine Zirkonoxidkeramik aufgetragen wird, erhöht sich die Lichtdurchlässigkeit der dentalen Restauration, d.h. deren Opazität wird verringert. Die Färbelösung eignet sich daher nicht zur Verhinderung, dass dunkle Materialien durch eine dentale Zirkonoxid-Restauration sichtbar sind.

Die EP 3 575 277 A1 beschreibt eine flüssige Zusammensetzung zum Erhöhen der Opazität einer dentalen Zirkonoxid-Keramik. Die Opaker-Zusammensetzung enthält eine wasserlösliche Aluminium- oder Lanthan-Verbindung, Wasser und ein organisches Lösungsmittel. Es ist jedoch z.B. aus der EP 3 558 671 A1 bekannt, dass die Dotierung mit Al bzw. La die lokalen Sintereigenschaften verändert. Daher ist es wünschenswert, die Verwendung von Al- und La-haltigen Opaker-Verbindungen zu vermeiden, um einen geringen Sinterverzug und damit eine hohe Passgenauigkeit der hergestellten dentalen Zirkonoxid-Keramik zu gewährleisten.

Die WO 2019/146908 offenbart eine Weißmittel-Zusammensetzung, dessen Anwendung auf Zirkonoxid-Dentalkeramiken auch zu einer Erhöhung der Opazität führt. Das Weißmittel enthält Metalloxid-Nanopartikel und/oder eine Metallionen-haltige Komponente sowie ein Stabilisierungsmittel. Die Verwendung von Metalloxid-Nanopartikeln ist allerdings aufwendig, da sie mehrstufige Verarbeitungsprozesse erfordert, und birgt darüber hinaus für den Verwender wegen des Einsatzes von Flusssäure hohe gesundheitliche Risiken.

Die WO 2015/148215 A1 offenbart eine Phosphor-haltige Zusammensetzung zum Erhöhen der Opazität von Zirkonoxid-Keramiken. Es ist jedoch bekannt, dass die Behandlung von ZirkonoxidMaterialien mit Phosphor-haltigen Zusammensetzungen, wie z.B. Phosphor-haltigen Säuren, nachteilig ist, weil dies einen negativen Einfluss auf den Verbund zu Adhäsiven hat, wie z.B. zu Phosphat-haltigen Zementen. Um einen belastbaren Verbund zwischen der Innenseite der dentalen Restauration und dem Zahnstumpf zu gewährleisten, ist daher das Aufbringen von Phosphat-haltigen Zusammensetzungen auf die Innenseite der dentalen Restauration, d.h. in deren Lumen, möglichst zu vermeiden. Allerdings wäre gerade dort die opakierende Wirkung wünschenswert, um die Sichtbarkeit von verfärbten Zahnstümpfen zu verringern.

Der Erfindung liegt die Aufgabe zugrunde, ein Mittel zur Verfügung zu stellen, mit dem die Opazität von Zirkonoxidkeramiken erhöht werden kann. Eine mit diesem Mittel behandelte Zirkonoxidkeramik sollte nicht nur vorteilhafte Eigenschaften in Bezug auf die ästhetische Erscheinung, sondern auch hinsichtlich der mechanischen Eigenschaften und der Passgenauigkeit aufweisen. Das Mittel sollte ferner einen Einsatz auch in dünnwandigen dentalen Restaurationen ermöglichen. Darüber hinaus sollte das Mittel gesundheitlich unbedenklich sein und eine unkomplizierte Verarbeitung ermöglichen. Außerdem sollte das Mittel eine hohe Stabilität und langanhaltende Homogenität bei Lagerung aufweisen.

Diese Aufgabe wird überraschenderweise durch die dentale Opaker-Zusammensetzung gemäß den Ansprüchen 1 bis 13 gelöst. Die Erfindung betrifft außerdem das Verfahren zur Herstellung einer dentalen Restauration nach den Ansprüchen 14 bis 18 sowie die Verwendung nach Anspruch 19.

Die erfindungsgemäße dentale Opaker-Zusammensetzung zeichnet sich dadurch aus, dass sie
(a) Alkalisilikat und
(b) Wasser
enthält, wobei das Alkalisilikat zumindest teilweise in dem Wasser gelöst ist.

Es ist überraschenderweise festgestellt worden, dass mit der erfindungsgemäßen Zusammensetzung auch ohne den Einsatz von opakierenden Metallionen, wie z.B. Ionen von Al, La, Zr und/oder Ti, oder Metalloxid-Nanopartikeln, die z.B. Al, Ti und/oder Zr enthalten, eine hohe Opazität in Oxidkeramiken, insbesondere Zirkonoxidkeramiken, bewirkt werden kann.

Die opakierende Wirkung der Zusammensetzung kann auf oberflächennahe Bereiche der Oxidkeramik begrenzt werden, wodurch es möglich ist, die gewünschten ästhetischen Effekte zur Abdeckung von verfärbten Zahnstümpfen oder dunklen Implantaten auch in dünnwandigen hochästhetischen dentalen Restaurationen zu erzielen.

Darüber hinaus ermöglicht es die erfindungsgemäße Zusammensetzung überraschenderweise, dentale Restaurationen herzustellen, die neben den gewünschten ästhetischen Eigenschaften auch besonders vorteilhafte mechanische Eigenschaften aufweisen. Es können dentale Restaurationen mit den gewünschten Opazitätseigenschaften erhalten werden, die sich durch eine Festigkeit und eine Langzeitstabilität im Bereich von Oxidkeramiken, insbesondere Zirkonoxidkeramiken, auszeichnen, die nicht mit der Opaker-Zusammensetzung behandelt wurden.

Ferner können mithilfe der erfindungsgemäßen Zusammensetzung auch dentale Restaurationen erhalten werden, die sich nicht nur durch die gewünschten Opazitätseigenschaften, sondern auch durch eine hohe Passgenauigkeit auszeichnen. Mit der Zusammensetzung behandelte Oxidkeramiken weisen nämlich beim abschließenden Dichtsintern ein vorteilhaftes Sinterverhalten mit geringem Sinterverzug auf. Es können daher dentale Restaurationen mit den gewünschten ästhetischen Eigenschaften erhalten werden, die auch ohne einen zusätzlichen Schritt zur Anpassung der Passform an den präparierten Stumpf oder die Implantat-Schnittstelle in den Mund des Patienten eingebracht werden können.

Als "Opaker-Zusammensetzung" wird im Sinne der vorliegenden Erfindung eine Zusammensetzung bezeichnet, die geeignet ist, die Opazität einer Oxidkeramik, insbesondere einer Zirkonoxidkeramik, zu erhöhen. Dabei tritt die Erhöhung der Opazität insbesondere auf, nachdem die Oxidkeramik mit der Zusammensetzung in Kontakt gebracht wurde und zusätzlich einer Wärmebehandlung unterzogen wurde. Die Erhöhung der Opazität erfolgt insbesondere in der Oxidkeramik selbst und nicht durch das Auftragen einer opaken Schicht auf die Oxidkeramik. Die Opaker-Zusammensetzung eignet sich insbesondere zum Erhöhen der Opazität in einer vorgesinterten Zirkonoxidkeramik, die bevorzugt aus mit Y₂O₃ stabilisierten tetragonalen Polykristallen hergestellt ist.

Die Opazität ist die Lichtundurchlässigkeit eines Materials und verhält sich reziprok zur Transluzenz, d.h. zur Lichtdurchlässigkeit eines Materials. Die Transluzenz ist das Verhältnis von durchgelassener zu einfallender Lichtintensität. Als Maß für die Opazität bzw. Transluzenz kann der Kontrastwertes (CR-Wert) gemäß dem British Standard 5612 bestimmt werden. Eine erfindungsgemäß bewirkte Erhöhung der Opazität liegt vor, wenn in einem mit der Opaker-Zusammensetzung behandelten und gegebenenfalls wärmebehandelten Teil einer Oxidkeramik, insbesondere Zirkonoxidkeramik, wie z.B. einer dichtgesinterten dentalen Restauration, die Opazität gegenüber einem anderen unbehandelten Teil der Oxidkeramik erhöht ist. Sollte die gesamte Oxidkeramik mit der Zusammensetzung behandelt worden sein, kann eine auf entsprechende Weise hergestellte unbehandelte Oxidkeramik zur Ermittlung des Vergleichswerts herangezogen werden. Die Erhöhung der Opazität wird im Sinne der vorliegenden Anmeldung auch als "Opakieren" bezeichnet.

Die Begriffe "Farbe" und "gefärbt" beziehen sich im Sinne der Erfindung auf den Farbwert und/oder die Helligkeit eines Materials. Farbwerte und Helligkeiten können durch den L*a*b-Wert, insbesondere nach DIN EN ISO 11664-4, oder nach einem in der Dentalindustrie gängigen Farbschlüssel bestimmt werden. Die Farbmessung kann mit handelsüblichen Messgeräten, wie einem Spektralphotometer CM-3700d (Konica Minolta) durchgeführt werden. Beispiele für Farbschlüssel sind der Vitapan classical^{®} und der Vita 3D Master^{®}, beide von der VITA Zahnfabrik H. Rauter GmbH & Co. KG, und der Chromascop^{®} der Ivoclar Vivadent AG.

Ohne Beschränkung auf eine Theorie wird angenommen, dass sich in der Oxidkeramik nach dem Auftragen der Opaker-Zusammensetzung dünne Glasphasen an den Oxidkeramik-Körnern bilden können. Es wird angenommen, dass die Erhöhung der Opazität auf dem Unterschied der Brechungsindizes von Oxidkeramik und Glasphasen beruht.

Es ist bevorzugt, dass das Alkalisilikat aus Lithiumsilikaten, Natriumsilikaten, Kaliumsilikaten und deren Mischungen und insbesondere Natriumsilikaten ausgewählt ist.

Bevorzugt kann das Alkalisilikat in Form von Natronwasserglas, Kaliwasserglas oder Lithiumwasserglas und insbesondere Natronwasserglas vorliegen. Als Natronwasserglas wird im Sinne der vorliegenden Erfindung Wasserglas bezeichnet, das Natriumsilikate und gegebenenfalls weitere Alkalisilikate enthält, wobei Natriumsilikate den größten Gewichtsanteil der Silikate ausmachen. Entsprechend weisen Kaliwasserglas und Lithiumwasserglas einen überwiegenden Gewichtsanteil an Kaliumsilikaten bzw. Lithiumsilikaten auf. Vorzugsweise beträgt die Dichte des Natronwasserglases 1,34 bis 1,38 g/cm³, die Dichte des Kaliumwasserglases 1,23 bis 1,27 g/cm³, insbesondere 1,25 g/cm³, und die Dichte des Lithiumwasserglases 1,16 bis 1,20 g/cm³, insbesondere 1,18 g/cm³.

In einer bevorzugten Ausführungsform enthält die Zusammensetzung 0,5 bis 37 Gew.-%, vorzugsweise 0,5 bis 20 Gew.-%, besonders bevorzugt 1 bis 10 Gew.-% Alkalisilikat.

In der Opaker-Zusammensetzung vorhandene Alkalisilikate können z.B. durch Röntgenfluoreszenzanalyse (XRF), Si-NMR, ¹³C-NMR, Atomabsorptionsspektrometrie (AAS) oder Ramanspektroskopie qualitativ nachgewiesen oder quantitativ bestimmt werden, gegebenenfalls in Kombination mit weiteren indirekten Analysen wie pH-Messungen, Glührückstandsanalysen oder rasterelektronenmikroskopischen Analysen von getrockneter oder auskristallisierter Opaker-Zusammensetzung.

In einer weiteren bevorzugten Ausführungsform enthält die Zusammensetzung 0,001 bis 10 Gew.-%, vorzugsweise 0,001 bis 8 Gew.-%, insbesondere 0,001 bis 5 Gew.-% Si, berechnet als SiO₂, und 0,0001 bis 10 Gew.-%, vorzugsweise 0,001 bis 8 Gew.-%, insbesondere 0,01 bis 5 Gew.-% Alkalimetall, berechnet als Alkalimetalloxid.

Ferner ist bevorzugt, dass die Zusammensetzung 63 bis 99,5 Gew.-%, vorzugsweise 80 bis 99,5 Gew.-%, besonders bevorzugt 90 bis 99,0 Gew.-% Wasser enthält.

In einer weiteren bevorzugten Ausführungsform liegt das Gewichtsverhältnis von Alkalisilikat zu Wasser in der Zusammensetzung im Bereich von 1:200 bis 1:6, insbesondere 1:100 bis 1:8.

Es ist weiter bevorzugt, dass das Alkalisilikat vollständig in dem Wasser gelöst ist.

In einer weiteren bevorzugten Ausführungsform liegt das Alkalisilikat in Form von Wasserglas vor, bei dem das Gewichtsverhältnis von Si, berechnet als SiO₂, zu Alkalimetall, berechnet als Alkalimetalloxid, 1 bis 6, vorzugsweise 1,5 bis 6 und besonders bevorzugt 2 bis 6 beträgt.

In einer weiteren bevorzugten Ausführungsform enthält die erfindungsgemäße Zusammensetzung ein Verdickungsmittel, vorzugsweise ein organisches Verdickungsmittel.

Unter einem Verdickungsmittel wird ein Mittel verstanden, durch das die Viskosität einer Lösung, vorzugsweise einer wässrigen Lösung, erhöht werden kann.

Es wurde festgestellt, dass durch die Zugabe eines Verdickungsmittels beeinflusst werden kann, wie tief die Opaker-Zusammensetzung in die Zirkonoxidkeramik eindringt. Dies ist insbesondere dann vorteilhaft, wenn die opakierende Wirkung der Zusammensetzung auf oberflächennahe Bereiche begrenzt werden soll. Auf diese Weise kann auch in dünnwandigen dentalen Restaurationen eine Abdeckung verfärbter Stümpfe oder dunkler Implantate erzielt werden, die nicht mit unerwünschten ästhetischen Effekten auf der Außenseite der Restauration einhergeht.

In einer bevorzugten Ausführungsform ist das Verdickungsmittel ein Polysaccharid. Es ist ferner bevorzugt, dass das Verdickungsmittel ausgewählt ist aus der Gruppe bestehend aus Polyvinylpyrrolidon, Polyethylenglykol, Polypropylenglykol, Hydroxyethylcellulose, Ethylcellulose, Methylcellulose, Xanthan, Carbomer, Pektinen, Kieselsol, Stärke, Gelatine, Alginaten und deren Mischungen und besonders bevorzugt Polyvinylpyrrolidon, Polyethylenglykol oder deren Mischung ist. Ganz besonders bevorzugt ist das Verdickungsmittel Polyvinylpyrrolidon.

Es ist bevorzugt, dass das Polyvinylpyrrolidon ein Molekulargewicht im Bereich von 10.000 bis 1.000.000 g/mol und das Polyethylenglykol ein Molekulargewicht im Bereich von 200 bis 100.000 g/mol, bezogen jeweils auf das Gewichtsmittel des Molekulargewichts, aufweisen.

Ganz besonders bevorzugt ist Polyvinylpyrrolidon mit einem Gewichtsmittel des Molekulargewichts von 10.000 bis 500.000 g/mol, insbesondere 10.000 bis 100.000 g/mol.

Es wurde festgestellt, dass durch die Verwendung von Polyvinylpyrrolidon die Herstellung der erfindungsgemäßen Zusammensetzung erheblich erleichtert wird. Insbesondere ist es mit Polyvinylpyrrolidon möglich, schneller die Zusammensetzungen herzustellen, klare Lösungen zu erhalten und es kann ferner auf aufwendige Lösungs- und Mischverfahren, wie z.B. die Verwendung eines Ultraschallbads, verzichtet werden.

Die Verwendung von Polyvinylpyrrolidon hat sich auch als vorteilhaft in Bezug auf die Stabilität der erfindungsgemäßen Zusammensetzung erwiesen. Nachteilige Lagerungseffekte der Zusammensetzung, wie z.B. eine Phasentrennung oder Ausfällung, können durch die Verwendung von Polyvinylpyrrolidon vermieden werden.

Es hat sich insbesondere gezeigt, dass eine Zusammensetzung mit Polyvinylpyrrolidon im Vergleich zu Zusammensetzungen, die andere Verdickungsmittel enthalten, weniger zur Bildung von unerwünschten Trübungen und Ausflockungen neigt. Da solche Trübungen und Ausflockungen mit bloßem Auge sichtbar sind, kann deren Bildung beispielsweise durch Beobachtung der Zusammensetzung verfolgt werden.

Besonders bevorzugt ist Polyethylenglykol mit einem Gewichtsmittel des Molekulargewichts von 200 bis 50.000 g/mol, insbesondere 200 bis 35.000 g/mol.

Es ist bevorzugt, dass die Zusammensetzung 0 bis 50 Gew.-%, vorzugsweise 0,1 bis 50 Gew.-%, insbesondere 0,2 bis 30 Gew.-%, besonders bevorzugt 0,5 bis 20 Gew.-% Verdickungsmittel enthält.

In einer bevorzugten Ausführungsform enthält die Zusammensetzung 0 bis 30 Gew.-%, vorzugsweise 0,1 bis 30 Gew.-%, insbesondere 0,2 bis 20 Gew.-%, besonders bevorzugt 0,5 bis 10 Gew.-%, ganz besonders bevorzugt 0,5 bis 6 Gew.-% Polyvinylpyrrolidon.

In einer weiteren bevorzugten Ausführungsform liegt das Gewichtsverhältnis von Verdickungsmittel, insbesondere Polyvinylpyrrolidon, zu Wasser in der Zusammensetzung im Bereich von 1:200 bis 1:10, insbesondere 1:100 bis 1:12, besonders bevorzugt 1:50 bis 1:15.

Es ist auch bevorzugt, dass das Gewichtsverhältnis von Verdickungsmittel, insbesondere Polyvinylpyrrolidon, zu Alkalisilikat in der Zusammensetzung im Bereich von 1:20 bis 5:1, insbesondere 1:15 bis 1:4 beträgt.

In einer weiteren bevorzugten Ausführungsform enthält die Zusammensetzung 0 bis 50 Gew.-%, vorzugsweise 0,1 bis 50 Gew.-%, insbesondere 0,2 bis 30 Gew.-%, besonders bevorzugt 0,5 bis 20 Gew.-% Polyethylenglykol.

Es hat sich gezeigt, dass die Eindringtiefe der Opaker-Zusammensetzung durch den Anteil an Verdickungsmittel, wie z.B. Polyvinylpyrrolidon und Polyethylenglykol, sowie die Eigenschaften des Verdickungsmittels beeinflusst werden kann. In der Regel führt beispielsweise ein hoher Anteil an Polyvinylpyrrolidon oder Polyethylenglykol ebenso wie ein hohes Molekulargewicht dieser Bestandteile zu einer geringen Eindringtiefe, womit die opakierende Wirkung für den Betrachter abgeschwächt werden kann.

In einer bevorzugten Ausführungsform enthält die erfindungsgemäße Opaker-Zusammensetzung ferner ein organisches Lösungsmittel, vorzugsweise in einer Menge von 0,5 bis 30 Gew.-%, insbesondere 1 bis 20 Gew.-%. Geeignete organische Lösungsmittel sind insbesondere mit Wasser mischbar und ausgewählt aus der Gruppe bestehend aus Methanol, Ethanol, Isopropanol, Glycerin, Ethylenglykol, Aceton, 1,4-Dioxan und deren Mischungen. Aus der Zugabe von organischen Lösungsmitteln können sich z.B. vorteilhafte Benetzungs- und Trocknungseigenschaften der Opaker-Zusammensetzung ergeben.

In einer bevorzugten Ausführungsform ist die Zusammensetzung im Wesentlichen frei von Metalloxid-Nanopartikeln. Es ist ferner bevorzugt, dass die Zusammensetzung im Wesentlichen frei von Al, La, Zr, P und Ti ist.

Es ist ferner bevorzugt, dass die Opaker-Zusammensetzung eine Kennfarbe enthält. Eine Kennfarbe ist im Sinne der vorliegenden Erfindung ein Mittel, das in der Zusammensetzung eine farbgebende Wirkung aufweist, ohne einen wesentlichen Einfluss auf die Farbe der dichtgesinterten Oxidkeramik, insbesondere Zirkonoxidkeramik, zu haben. Bevorzugte Kennfarben sind organische Farbstoffe, insbesondere Triphenylmethanfarbstoffe, wie z.B. Patentblau V (E131). Durch die Kennfarbe kann die Sichtbarkeit der erfindungsgemäßen Zusammensetzung auf der vorgesinterten Oxidkeramik verbessert und dadurch das präzise Auftragen der Zusammensetzung erleichtert werden.

Es ist ferner bevorzugt, dass die erfindungsgemäße Zusammensetzung in Form einer Flüssigkeit und insbesondere in Form einer Lösung vorliegt. Dadurch kann ein einfaches Auftragen auf die Oxidkeramik, z.B. mithilfe eines Pinsels oder eines geeigneten Applikators, gewährleistet werden. Besonders bevorzugt liegt die Zusammensetzung in Form einer homogenen Flüssigkeit vor, um eine gleichmäßige opakierende Wirkung sicherzustellen.

Es ist besonders bevorzugt, dass die Zusammensetzung einen pH-Wert im Bereich von 7,5 bis 12,5, vorzugsweise 8,5 bis 12,5, besonders bevorzugt 9,5 bis 12 aufweist. Auf diese Weise wird das Risiko für saure oder alkalische Verätzungen für Verwender der Opaker-Zusammensetzung reduziert. Es ergibt sich insbesondere eine Verbesserung der Arbeitssicherheit für Zahntechniker und Zahnärzte gegenüber den aus dem Stand der Technik bekannten Opaker-Zusammensetzungen mit einem pH-Wert über 12,5.

In einer weiteren bevorzugten Ausführungsform weist die Zusammensetzung bei Raumtemperatur, d.h. bei einer Temperatur von 23°C, eine Viskosität im Bereich von 1 bis 5.000, vorzugsweise 1 bis 1.000, besonders bevorzugt 1 bis 100 mPa*s im Scherratebereich von 1 bis 100 s⁻¹ auf. Die Viskosität kann beispielsweise mit einem Rheometer vom Typ MCR302 der Anton Paar GmbH und unter Verwendung eines CP50 Messsystems (Kegel-Platte mit 50 mm Durchmesser) bestimmt werden. Typischerweise weisen Zusammensetzungen mit einer Viskosität innerhalb der genannten Bereiche besonders vorteilhafte Eigenschaften in Bezug auf die Verarbeitbarkeit auf.

Die Erfindung betrifft ferner ein Verfahren zur Herstellung einer dentalen Restauration, bei dem in mindestens einem Teil einer Oxidkeramik, insbesondere einer Zirkonoxidkeramik, die Opazität erhöht wird, indem eine vorstehend beschriebene erfindungsgemäße Zusammensetzung auf diesen Teil aufgebracht und einer Wärmebehandlung unterzogen wird.

Bevorzugt erfolgt die Wärmebehandlung für mindestens 1 min, insbesondere mindestens 10 min bei einer Temperatur von vorzugsweise 1000 bis 1700°C, insbesondere 1000°C bis 1600°C, besonders bevorzugt 1000 bis 1500°C.

Es ist bevorzugt, dass die Zusammensetzung bis zu einer Tiefe von 1 bis 2000 µm, insbesondere 2 bis 1000 µm und besonders bevorzugt 5 bis 500 µm in die Oxidkeramik eindringt. Die Eindringtiefe kann dadurch bestimmt werden, dass die Oxidkeramik mit der Zusammensetzung behandelt, der Wärmebehandlung unterzogen und danach beispielsweise mithilfe einer Säge geteilt wird und dann an der durch das Teilen zugänglich gemachten Oberfläche mittels eines Mikroskops (z.B. Olympus SZX10) gemessen wird, wie weit die Zusammensetzung in die Oxidkeramik eingedrungen ist.

Es ist ferner bevorzugt, die Opaker-Zusammensetzung im Lumen der herzustellenden dentalen Restauration aufzutragen.

In einer bevorzugten Ausführungsform wird die Opazität in mindestens einem Teil einer Oxidkeramik erhöht, auf den zuvor ein Sperrmittel aufgetragen und gegebenenfalls getrocknet wurde. Unter einem Sperrmittel wird ein Mittel verstanden, das nach Aufbringung und gegebenenfalls Trocknung auf eine Oxidkeramik die Eindringtiefe der Opaker-Zusammensetzung in der Oxidkeramik verringern kann. In der Regel bildet das Sperrmittel in der Oxidkeramik eine Sperrschicht, die dem Eindringen der Opaker-Zusammensetzung entgegenwirkt.

In einer bevorzugten Ausführungsform enthält das Sperrmittel ein Lösungsmittel, insbesondere ein organisches Lösungsmittel, und ein darin dispergiertes Polymer, insbesondere ein Verdickungsmittel, sowie gegebenenfalls eine Kennfarbe. Geeignete Verdickungsmittel sind die für die Opaker-Zusammensetzung genannten Verdickungsmittel.

In einer weiteren bevorzugten Ausführungsform wird in mindestens einem Bereich des Teils der Oxidkeramik, auf den die Opaker-Zusammensetzung aufgebracht wurde, dieselbe Zusammensetzung erneut oder eine von dieser verschiedene erfindungsgemäße Zusammensetzung aufgebracht. Vorzugsweise wird die Zusammensetzung vor dem wiederholten Auftragen zunächst getrocknet, bevor erneut dieselbe oder eine von dieser verschiedene erfindungsgemäße Zusammensetzung aufgebracht wird. Durch das mehrfache Aufbringen der Opaker-Zusammensetzung, gegebenenfalls in Kombination mit der Wahl von Zusammensetzungen mit unterschiedlichen Eigenschaften, wie z.B. unterschiedlichen Eindringtiefen, können auch komplizierte dreidimensionale Opazitätsverläufe erzeugt werden.

Die Oxidkeramik, insbesondere Zirkonoxidkeramik, kann beim Aufbringen der erfindungsgemäßen Zusammensetzung in ungesintertem oder vorgesintertem Zustand vorliegen. Es ist besonders bevorzugt, dass die Oxidkeramik vorgesintert ist. Die vorgesinterte Oxidkeramik liegt typischerweise in einem offenporigen Zustand mit einer Dichte von vorzugsweise 30 bis 70%, insbesondere 40 bis 65%, der Dichte der dichtgesinterten Oxidkeramik vor. Typischerweise geht eine geringere Dichte der vorgesinterten Oxidkeramik mit einer größeren Eindringtiefe der Opaker-Zusammensetzung einher.

In einer besonders bevorzugten Ausführungsform geht die Wärmebehandlung mit dem Dichtsintern der Oxidkeramik einher.

Typischerweise entsteht durch das Dichtsintern eine dentale Restauration mit den gewünschten Eigenschaften. Es hat sich gezeigt, dass Oxidkeramiken, insbesondere Zirkonoxidkeramiken, in denen die Opazität mittels der erfindungsgemäßen Zusammensetzung erhöht wurde, ein vorteilhaftes Sinterverhalten mit geringem Sinterverzug aufweisen.

Durch das erfindungsgemäße Verfahren können dentale Restaurationen mit vorteilhaften mechanischen Eigenschaften hergestellt werden.

Bevorzugt weist eine nach dem erfindungsgemäßen Verfahren hergestellte dichtgesinterte dentale Restauration eine biaxiale Bruchfestigkeit von mindestens 500 MPa, insbesondere mindestens 600 MPa und besonders bevorzugt mindestens 700 MPa, bestimmt nach ISO 6872:2015, auf.

Es ist auch bevorzugt, dass eine nach dem erfindungsgemäßen Verfahren hergestellte dichtgesinterte dentale Restauration eine biaxiale Bruchfestigkeit von mindestens 70%, vorzugsweise mindestens 80%, insbesondere mindestens 95% aufweist, bestimmt nach ISO 6872:2015 und bezogen jeweils auf die biaxiale Bruchfestigkeit einer entsprechenden dichtgesinterten dentale Restauration, die nicht mit der Opaker-Zusammensetzung behandelt wurde.

Es ist ferner bevorzugt, dass eine nach dem erfindungsgemäßen Verfahren hergestellte dichtgesinterte dentale Restauration eine hohe Langzeitstabilität aufweist. Besonders bevorzugt zeichnet sich die dichtgesinterte dentale Restauration durch ein Bestehen eines Ermüdungstests aus, bei dem die dentale Restauration für 2.000.000 Prüfzyklen mit mindestens 450 N, vorzugsweise mindestens 550 N, besonders bevorzugt mindestens 650 N, unter Verwendung einer servopneumatischen Zwei-Säulen-Tischprüfmaschine 5 kN der DYNA-MESS Prüfsysteme GmbH mit einer sinusförmigen Frequenz (Vorlast ist 10% der Hauptlast) von 5 Hz in 37°C warmem Wasser belastet wird.

Die nach dem erfindungsgemäßen Verfahren hergestellten dentalen Restaurationen können durch alle bekannten Verfahren, z.B. durch Zementieren, Kleben oder Schrauben, auf einem Stumpf oder Implantatgerüst befestigt werden.

Aus dem Stand der Technik sind chemische und mechanische Verfahren zur Vorbehandlung der Oberfläche von Zirkonoxid bekannt, um die Verbindung zur Stumpf- oder Implantatoberfläche zu verstärken.

In einer bevorzugten Ausführungsform des Verfahrens erfolgt in dem Teil der Oxidkeramik, in dem die Opazität der Oxidkeramik erhöht wurde, eine Behandlung mit einer Ätzflüssigkeit. Die Behandlung kann in einem Teilbereich des opakierten Teils erfolgen und kann sich auch auf nicht opakierte Teile der Oxidkeramik erstrecken. Es hat sich überraschenderweise gezeigt, dass die Behandlung mit einer Ätzflüssigkeit auch in den mit der Opaker-Zusammensetzung behandelten Bereichen zu einer Verbesserung des Verbundes mit dem Stumpf oder Implantat führen kann.

In einer besonders bevorzugten Ausführungsform enthält die Ätzflüssigkeit Flusssäure.

In einer weiteren bevorzugten Ausführungsform des Verfahrens ist die dentale Restauration eine Brücke, ein Inlay, ein Onlay, ein Veneer, ein Abutment, eine Teilkrone, eine Krone oder eine Schale.

Die Erfindung betrifft ferner die Verwendung der erfindungsgemäßen dentalen Opaker-Zusammensetzung zur Erhöhung der Opazität in einer Oxidkeramik, insbesondere Zirkonoxidkeramik, bei der Herstellung einer dentalen Restauration.

Alle stofflichen Definitionen, Verfahrensschritte und Verfahrensparameter, die im Rahmen der erfindungsgemäßen Opaker-Zusammensetzung und des erfindungsgemäßen Verfahrens beschrieben wurden, sind auch für diese erfindungsgemäße Verwendung geeignet.

Die Erfindung wird im Folgenden anhand von Ausführungsbeispielen näher erläutert.

### Beispiele

### Beispiele 1 und 2: Erfindungsgemäße Opaker-Zusammensetzungen

Es wurden Lösungen mit den chemischen Zusammensetzungen gemäß Tabelle 1 hergestellt.

**Tabelle 1**

| **Beispiel** | **1** | **2** |
|---|---|---|
| *Bestandteil* | *Gewicht (g)* | *Gewicht (g)* |
| H₂O | 21,37 | 20,00 |
| Polyvinylpyrrolidon K30 | 1,125 | 1,00 |
| Natronwasserglas (33,6 bis 37 Gew.-%-ige Lsg.) | 2,55 | 1,05 |

Dabei wurde zur Herstellung dieser und der nachfolgenden Beispiele Natronwasserglas der Reinheitsklasse "reinst" der Carl Roth GmbH, das laut Herstellerangaben neben Natriumsilikat keine weiteren Alkalisilikate enthält, sowie Polyvinylpyrrolidon (PVP) K30 der Reinheitsklasse "reinst" der BASF SE, das unter dem Namen Luviskol^{®} K30 vertrieben wird, verwendet. Luviskol^{®} K30 besteht gemäß Herstellerangaben aus Polyvinylpyrrolidon mit einem K-Wert (nach Fikentscher, 1% in Wasser) zwischen 27.0 und 33.0 und einem Gewichtsmittel des Molekulargewichts von etwa 49.000 g/mol. Der in der Tabelle 1 und allen nachfolgenden Tabellen und Zusammensetzungen für die jeweiligen Bestandteile angegebene Gehalt in Gew.-% entspricht der Herstellerangabe.

Zwei vorgesinterte Zirkonoxidkeramiken in Form von dentalen Kronen wurden bereitgestellt. Die Lösungen der Beispiele 1 und 2 wurden mit einem Pinsel auf die Innenseite von je einer Krone aufgetragen und an der Luft getrocknet. Anschließend wurden die Kronen für 120 min bei 1500°C in einem Ofen des Typs Programat S1-1600 der Ivoclar Vivadent AG dichtgesintert.

Nach dem Dichtsintern wurde bei beiden Kronen in den mit den Lösungen behandelten Oberflächenbereichen eine Opazität beobachtet, die im Vergleich zu den nicht mit den Lösungen behandelten Bereichen erhöht war.

### Beispiele 3 bis 5: Opaker-Zusammensetzungen mit PVP K30, K90 und K360

Es wurden Opaker-Zusammensetzungen mit den chemischen Zusammensetzungen gemäß Tabelle 2 hergestellt.

**Tabelle 2**

| **Beispiel** | **3** | **4** | **5** |
|---|---|---|---|
| *Bestandteil* | *Gewicht (g)* | *Gewicht (g)* | *Gewicht (g)* |
| H₂O | 21,37 | 21,37 | 21,37 |
| Polyvinylpyrrolidon K30 | 1,125 | - | - |
| Polyvinylpyrrolidon K90 | - | 1,125 | - |
| Polyvinylpyrrolidon K360 | - | - | 1,125 |
| Natronwasserglas (33,6 bis 37 Gew.%-ige Lsg.) | 2,5 | 2,5 | 2,5 |

Es wurden drei vorgesinterte Zirkonoxidkeramiken in Form von dentalen Kronen bereitgestellt, dann mit den Opaker-Zusammensetzungen der Beispiele 3 bis 5 behandelt und einer Wärmebehandlung nach dem für die Beispiele 1 und 2 beschriebenen Verfahren unterzogen.

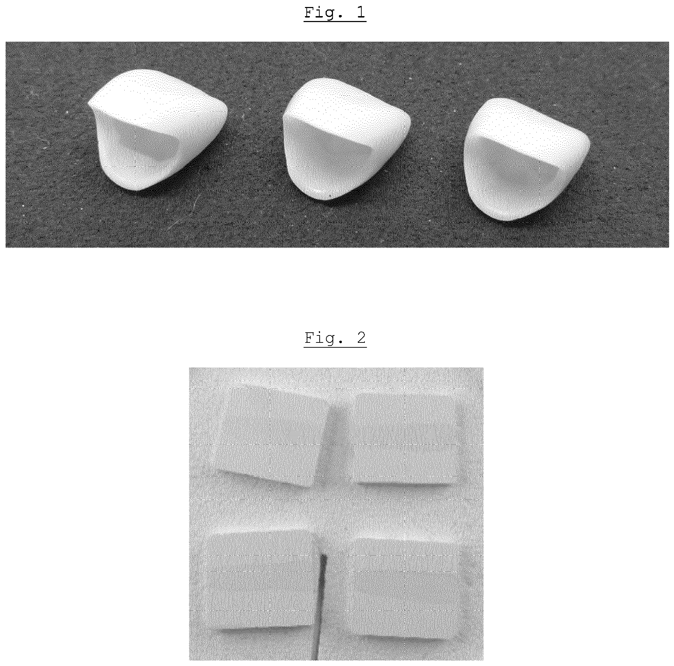

Die dichtgesinterten Zirkonoxidkeramiken sind in Figur 1 gezeigt, dabei ist die Zirkonoxidkeramik links mit der Opaker-Zusammensetzung von Beispiel 3, die Zirkonoxidkeramik in der Mitte mit der Opaker-Zusammensetzung von Beispiel 4 und die Zirkonoxidkeramik rechts mit der Opaker-Zusammensetzung von Beispiel 5 behandelt worden. Es lässt sich erkennen, dass für den Betrachter die opakierende Wirkung der Zusammensetzung nach Beispiel 3, d.h. unter Verwendung von Polyvinylpyrrolidon K30, am stärksten ist.

### Beispiel 6: Opaker-Zusammensetzungen mit Kennfarbe

Es wurde eine Lösung mit der chemischen Zusammensetzung gemäß Tabelle 3 hergestellt und in bis zu vier Schichten auf plättchenförmige vorgesinterte Zirkonoxidkeramiken aufgetragen.

**Tabelle 3**

| *Bestandteil* | *Gewicht (g)* |
|---|---|
| H₂O | 20 |
| Polyvinylpyrrolidon K30 | 1 |
| Natronwasserglas (33,6 bis 37 Gew.-%-ige Lsg.) | 1,05 |
| Patentblau V (E131) | <0,01 |

Die mit der Opaker-Zusammensetzung behandelten vorgesinterten Zirkonoxidkeramiken sind in Figur 2 abgebildet. Durch die Zugabe der Kennfarbe waren die mit der Opaker-Zusammensetzung behandelten Bereiche der Zirkonoxidkeramik deutlich von den unbehandelten Bereichen zu unterscheiden.

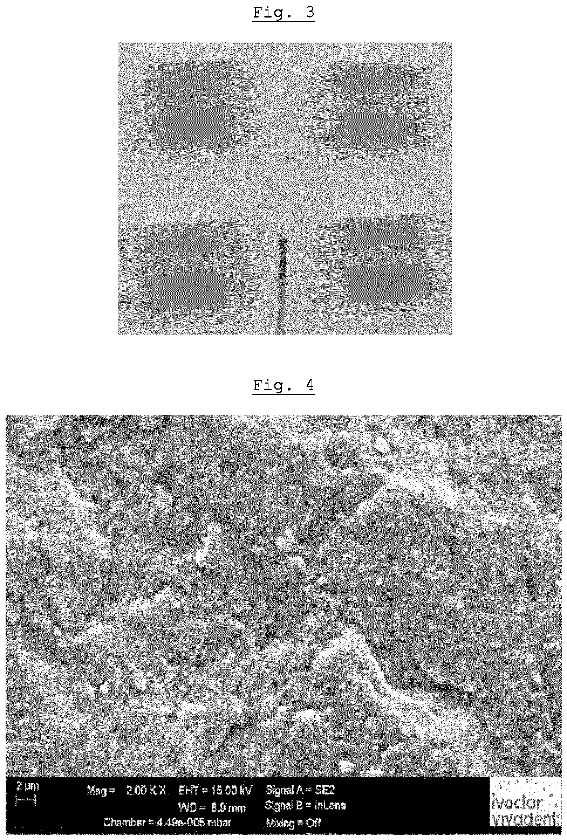

Die Zirkonoxidkeramiken wurden nach dem für die Beispiele 1 und 2 beschriebenen Verfahren dichtgesintert. Die Opazität der dichtgesinterten Zirkonoxidkeramiken war in den Bereichen, die mit der Opaker-Zusammensetzung behandelt worden waren, im Vergleich zu den unbehandelten Bereichen erhöht. Dies ist auch aus den in der Figur 3 abgebildeten Zirkonoxidkeramiken ersichtlich.

### Beispiel 7: Bestimmung der Eindringtiefe von Opaker-Lösungen

Es wurde eine plättchenförmige vorgesinterte Zirkonoxidkeramik bereitgestellt und mit der Opaker-Lösung mit der chemischen Zusammensetzung gemäß Tabelle 4 behandelt, indem ca. 3 µl Opaker-Lösung auf die Zirkonoxidkeramik gegeben wurden.

**Tabelle 4**

| *Bestandteil* | *Gewicht (g)* |
|---|---|
| H₂O | 9,9 |
| Natronwasserglas (33,6 bis 37 Gew.-%-ige Lsg.) | 0,1 |

Die Zirkonoxidkeramik wurde nach dem für die Beispiele 1 und 2 beschriebenen Verfahren dichtgesintert und dann zerteilt, um die Eindringtiefe der opakierenden Lösung bestimmen zu können. Die Messung mittels eines Mikroskops vom Typ Olympus SZX10 ergab eine Eindringtiefe von 0,14 mm.

### Beispiel 8: Analyse der Gefügestruktur mittels REM

Es wurde eine vorgesinterte Zirkonoxidkeramik mit der Opaker-Zusammensetzung nach Tabelle 5 behandelt.

**Tabelle 5**

| *Bestandteil* | *Gewicht (g)* |
|---|---|
| H₂O | 9 |
| Natronwasserglas (33,6 bis 37 Gew.-%ige Lsg.) | 1 |

Die Zirkonoxidkeramik wurde nach dem für die Beispiele 1 und 2 beschriebenen Verfahren dichtgesintert. Danach wurde die Gefügestruktur der Zirkonoxidkeramik mittels Rasterelektronenmikroskopie (REM) untersucht. Die Gefügestruktur eines Oberflächenbereichs, der mit der Zusammensetzung gemäß Tabelle 5 behandelt wurde, ist in Figur 4 abgebildet. Es ist zu erkennen, dass sich die Struktur der Zirkonoxidkeramik durch eine geringe Korngröße auszeichnet. Ferner sind keine Poren zu sehen.

### Beispiele 9 bis 11: Prüfkörper zur Bestimmung der biaxialen Bruchfestigkeit

Zur Bestimmung der biaxialen Bruchfestigkeit gemäß ISO 6872:2015 wurden scheibenförmige Prüfkörper mit einer Dicke von 1,2 mm und einem Durchmesser von 13,0 mm aus vorgesintertem Zirkonoxid hergestellt.

Auf die Prüfkörper der Beispiele 10 und 11 wurde ein Sperrmittel aufgetragen und getrocknet, dass 65,37 Gew.-% H₂O, 18,247 Gew.-% PVP K30, 16,343 Gew.-% Ethanol und 0,04 Gew.-% Patentblau V (E131) enthielt.

Es wurden drei Opaker-Lösungen mit chemischen Zusammensetzungen gemäß der Tabelle 6 hergestellt.

**Tabelle 6**

| **Beispiel** | **9** | **10** | **11** |
|---|---|---|---|
| *Bestandteil* | *Gewicht (g)* | *Gewicht (g)* | *Gewicht (g)* |
| H₂O | 9,9 | 9,5 | 9 |
| Natronwasserglas (33,6 bis 37 Gew.%-ige Lsg.) | 0,1 | 0,5 | 1 |

Es wurden jeweils fünf Prüfkörper mit den Opaker-Zusammensetzungen der Beispiele 9 bis 11 behandelt, indem auf jeden Prüfkörper 3 µl Opaker-Zusammensetzung aufgetragen wurden. Alle Prüfkörper wurden dichtgesintert, auf drei konzentrisch angeordneten Kugeln platziert und mittig bis zum Versagen belastet.

Die mittlere biaxiale Bruchfestigkeit der Prüfkörper der Beispiele 9 bis 11 ist in Tabelle 7 dargestellt.

**Tabelle 7**

| **Beispiel** | **Mittlere biaxiale Bruchfestigkeit (MPa)** |
|---|---|
| 9 | 988 |
| 10 | 947 |
| 11 | 899 |

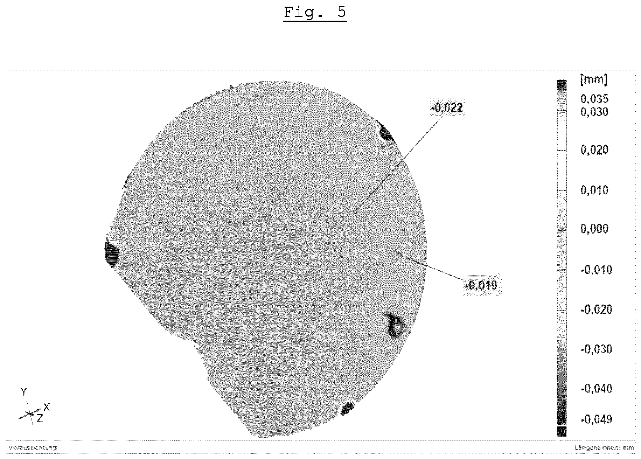

An den Prüfkörpern der Beispiele 9 bis 11 war nach dem Dichtsintern keine Deformation zu erkennen. Zur Veranschaulichung wurden die Proben einer 3D-Messung unter Verwendung eines optischen 3D-Scanners (Modell ATOS Capsule der Firma GOM GmbH) unterzogen. Ein beispielhaftes Ergebnis der 3D-Messung ist in Fig. 5 dargestellt.

### Beispiel 12: Ermüdungstest

Es wurden vier 3-gliedrige Seitenzahnbrücken aus Zirkonoxid (e.max ZirCAD der Ivoclar Vivadent AG) hergestellt. Die Brücken wiesen eine Wandstärke von 0,6 mm und Verbinderquerschnitte von etwa 3 mm x 3 mm auf.

Es wurde eine erfindungsgemäße Opaker-Zusammensetzung aus 21,37 g H₂O, 1,125 g PVP K30 und 2,5 g Natronwasserglas (33,6 bis 37 Gew.-%-ige Lsg.) hergestellt. Die Zusammensetzung wurde bei zwei Seitenzahnbrücken auf die gesamte Lumenoberfläche aufgetragen. Auf die Oberfläche der zwei verbleibenden Seitenzahnbrücken wurde keine Opaker-Zusammensetzung aufgetragen. Alle vier Seitenzahnbrücken wurden dichtgesintert.

Dann wurden die Seitenzahnbrücken auf Stümpfe aus Polymethylmethacrylat (PMMA) aufgebracht, indem die Innenseite der Seitenzahnbrücken und die Oberseiten der Stümpfe zunächst durch Sandstrahlen mechanisch aufgeraut wurden und anschließend mit einem selbsthärtenden Glasionomerzement (Vivaglass CEM der Ivoclar Vivadent AG) miteinander verbunden wurden.

Daraufhin wurden die Seitenzahnbrücken einem Ermüdungstest in einer servopneumatischen Zwei-Säulen-Tischprüfmaschine 5 kN der DYNA-MESS Prüfsysteme GmbH (Zwei-Säulen-Tischprüfmaschine 5 kN) unterzogen, bei dem die Seitenzahnbrücken für 2.000.000 Zyklen mit 550 N bei einer sinusförmigen Frequenz (Vorlast ist 10% der Hauptlast) von 5 Hz in 37°C warmem Wasser belastet wurden.

Sowohl die zwei mit der Opaker-Zusammensetzung behandelten Seitenzahnbrücken als auch die zwei Brücken, die nicht mit der Opaker-Zusammensetzung behandelt wurden, wiesen nach Abschluss des Ermüdungstests keine sichtbaren Schäden auf.

### Beispiele 13 und 14: Homogenität der Zusammensetzung

Es wurden zwei Opaker-Zusammensetzungen mit den chemischen Zusammensetzungen gemäß Tabelle 8 hergestellt. Dazu wurde erst das Natronwasserglas für etwa 15 min bei Raumtemperatur mit dem Wasser homogenisiert. Anschließend wurde das Verdickungsmittel hinzugefügt und ein weiterer Homogenisierungsschritt durchgeführt. Zur Homogenisierung wurde ein Magnetrührer mit einer Drehzahl von 400 bis 600 Umdrehungen pro Minute verwendet.

**Tabelle 8**

| **Beispiel** | **13** | **14** |
|---|---|---|
| *Bestandteil* | *Gewicht (g)* | *Gewicht (g)* |
| H₂O | 21,37 | 21,37 |
| Polyvinylpyrrolidon K30 | 1,125 | - |
| Polyethylenglykol 600 | - | 1,125 |
| Natronwasserglas (33,6 bis 37 Gew.%-ige Lsg.) | 2,5 | 2,5 |

Die Zusammensetzungen wurden direkt nach ihrer Herstellung zur Prüfung der Homogenität genau in Augenschein genommen. Dann wurden die Zusammensetzungen bei Raumtemperatur gelagert und über einen Zeitraum von mehreren Wochen regelmäßig erneut auf ihre Homogenität geprüft.

Für die Zusammensetzung von Beispiel 14 wurden bereits direkt nach deren Herstellung Ausflockungen beobachtet, und die Menge der gebildeten Ausflockungen wurde mit fortschreitender Lagerzeit ersichtlich größer. Die Ausflockungen setzten sich am Boden des Gefäßes ab.

Die Zusammensetzung von Beispiel 13 war hingegen direkt nach der Herstellung klar, und es wurden keine Ausflockungen beobachtet. Erst nach über zwei Wochen waren erste Ausflockungen sichtbar und die Ausflockungen bildeten sich deutlich langsamer als bei der Zusammensetzung von Beispiel 14.

## Patentansprüche

1. Dentale Opaker-Zusammensetzung, die
(a) Alkalisilikat und
(b) Wasser
enthält, wobei das Alkalisilikat zumindest teilweise in dem Wasser gelöst ist.

2. Zusammensetzung nach Anspruch 1, die ein Verdickungsmittel, vorzugsweise ein organisches Verdickungsmittel, enthält.

3. Zusammensetzung nach Anspruch 2, bei der das Verdickungsmittel ausgewählt ist aus der Gruppe bestehend aus Polyvinylpyrrolidon, Polyethylenglykol, Polypropylenglykol, Hydroxyethylcellulose, Ethylcellulose, Methylcellulose, Xanthan, Carbomer, Pektinen, Kieselsol, Stärke, Gelatine, Alginaten und deren Mischungen und besonders bevorzugt Polyvinylpyrrolidon, Polyethylenglykol oder deren Mischung ist.

4. Zusammensetzung nach einem der Ansprüche 1 bis 3, bei der das Alkalisilikat aus Lithiumsilikaten, Natriumsilikaten, Kaliumsilikaten und deren Mischungen und insbesondere Natriumsilikaten ausgewählt ist.

5. Zusammensetzung nach Anspruch 3 oder 4, bei der das Polyvinylpyrrolidon ein Molekulargewicht im Bereich von 10.000 bis 1.000.000 g/mol und das Polyethylenglykol ein Molekulargewicht im Bereich von 200 bis 100.000 g/mol, bezogen jeweils auf das Gewichtsmittel des Molekulargewichts, aufweisen.

6. Zusammensetzung nach einem der Ansprüche 1 bis 5, die 0,5 bis 37 Gew.-%, vorzugsweise 0,5 bis 20 Gew.-%, besonders bevorzugt 1 bis 10 Gew.-% Alkalisilikat enthält.

7. Zusammensetzung nach einem der Ansprüche 1 bis 6, die 63 bis 99,5 Gew.-%, vorzugsweise 80 bis 99,5 Gew.-%, besonders bevorzugt 90 bis 99,0 Gew.-% Wasser enthält.

8. Zusammensetzung nach einem der Ansprüche 2 bis 7, die 0 bis 50 Gew.-%, vorzugsweise 0,1 bis 50 Gew.-%, insbesondere 0,2 bis 30 Gew.-%, besonders bevorzugt 0,5 bis 20 Gew.-% Verdickungsmittel enthält.

9. Zusammensetzung nach einem der Ansprüche 3 bis 8, die 0 bis 30 Gew.-%, vorzugsweise 0,1 bis 30 Gew.-%, insbesondere 0,2 bis 20 Gew.-%, besonders bevorzugt 0,5 bis 10 Gew.-% Polyvinylpyrrolidon enthält.

10. Zusammensetzung nach einem der Ansprüche 3 bis 9, die 0 bis 50 Gew.-%, vorzugsweise 0,1 bis 50 Gew.-%, insbesondere 0,2 bis 30 Gew.-%, besonders bevorzugt 0,5 bis 20 Gew.-% Polyethylenglykol enthält.

11. Zusammensetzung nach einem der Ansprüche 1 bis 10, die in Form einer Flüssigkeit und insbesondere in Form einer Lösung vorliegt.

12. Zusammensetzung nach Anspruch 11, die einen pH-Wert im Bereich von 7,5 bis 12,5, vorzugsweise 8,5 bis 12,5, besonders bevorzugt 9,5 bis 12 aufweist.

13. Zusammensetzung nach einem der Ansprüche 1 bis 12 die eine Viskosität im Bereich von 1 bis 5.000, vorzugsweise 1 bis 1.000, besonders bevorzugt 1 bis 100 mPa*s im Scherratebereich von 1 bis 100 s⁻¹ aufweist.

14. Verfahren zur Herstellung einer dentalen Restauration, bei dem in mindestens einem Teil einer Oxidkeramik, insbesondere einer Zirkonoxidkeramik, die Opazität erhöht wird, indem eine Zusammensetzung gemäß einem der Ansprüche 1 bis 13 auf diesen Teil aufgebracht und einer Wärmebehandlung unterzogen wird.

15. Verfahren nach Anspruch 14, bei dem die Oxidkeramik vorgesintert ist.

16. Verfahren nach Anspruch 14 oder 15, bei dem die Wärmebehandlung mit dem Dichtsintern der Oxidkeramik einhergeht.

17. Verfahren nach einem der Ansprüche 14 bis 16, wobei in dem Teil der Oxidkeramik, in dem die Opazität erhöht wurde, eine Behandlung mit einer Ätzflüssigkeit erfolgt.

18. Verfahren nach einem der Ansprüche 14 bis 17, bei dem die dentale Restauration eine Brücke, ein Inlay, ein Onlay, ein Veneer, ein Abutment, eine Teilkrone, eine Krone oder eine Schale ist.

19. Verwendung der Zusammensetzung nach einem der Ansprüche 1 bis 13 zur Erhöhung der Opazität in einer Oxidkeramik, insbesondere Zirkonoxidkeramik, bei der Herstellung einer dentalen Restauration.
